# FASCICULE DE BREVET EUROPEEN

(11) **EP 2 683 248 B1**
(45) Date de publication et mention de la délivrance du brevet: **28.02.2018**
(21) Numéro de dépôt: 11743083.5
(22) Date de dépôt: 01.07.2011
(51) Int. Cl.: A21D 2/14, C12N 1/18, C12N 15/01, A21D 8/04, C12R 1/865

(54) **SOUCHES DE SACCHAROMYCES CEREVISIAE APTES À LA PRODUCTION DE LEVURES DE PANIFICATION OSMOTOLÉRANTES ET PRÉSENTANT UNE RÉSISTANCE INTRINSÈQUE AUX ACIDES ORGANIQUES FAIBLES, LEURS PROCÉDÉS DE PRÉPARATION ET APPLICATIONS**
SACCHAROMYCES CEREVISIAE-STÄMME ZUR HERSTELLUNG VON OSMOTOLERANTEN BACKHEFEN MIT INTRINSISCHEM WIDERSTAND GEGENÜBER SCHWACHEN ORGANISCHEN SÄUREN, VERFAHREN ZU IHRER HERSTELLUNG UND ANWENDUNGEN
SACCHAROMYCES CEREVISIAE STRAINS SUITABLE FOR THE PRODUCTION OF BAKER'S YEASTS WHICH ARE OSMOTOLERANT AND WHICH EXHIBIT INTRINSIC RESISTANCE TO WEAK ORGANIC ACIDS, METHODS FOR THE PREPARATION THEREOF AND USES

(30) Priorité: 18.02.2011 FR 1151354
(43) Date de publication de la demande: 15.01.2014
(73) Titulaire: Lesaffre et Compagnie, 75001 Paris (FR)
(72) Inventeur: LADRIERE, Jean-Marc, F-59269 Querenaing (FR); BARTOLUCCI, Jean-Charles, F-59350 Saint André-lez-Lille (FR); SUCHER, Fabienne, F-59700 Marcq-en-Baroeul (FR); THOMAS, Benoit, F-59520 Marquette-lez-Lille (FR)
(74) Mandataire: Cabinet Plasseraud
(86) Numéro de dépôt international: PCT/FR2011/051550
(87) Numéro de publication internationale: WO 2012/110711

(56) Documents cités:
- EP-A1- 0 645 094
- EP-A1- 1 559 322
- WO-A1-99/51746
- WO-A1-2009/056708
- GB-A- 2 115 833
- DATABASE WPI Week 200937 Thomson Scientific, London, GB; AN 2009-J12140 XP002659941, & CN 101 418 265 A (ANQI YEAST YILI CO LTD) 29 avril 2009 (2009-04-29)

## Description

### DEMANDE PARENTE

La présente demande revendique la priorité de la demande française numéro FR 1151354 déposée le 18 février 2011.

### DOMAINE DE L'INVENTION

La présente invention a trait au domaine des souches de *Saccharomyces cerevisiae* aptes à la production de levures de panification, à leurs procédés de préparation, aux levures de panification produites à partir de ces souches, aux pâtes boulangères les contenant ainsi qu'aux procédés et produits de panifications susceptibles d'êtres obtenus par la mise en oeuvre de ces pâtes. Plus spécialement la présente invention concerne dans son aspect le plus général des souches de *Saccharomyces cerevisiae,* dites industrielles, aptes à produire des levures de panification osmotolérantes et présentant une résistance intrinsèque aux acides organiques faibles.

### ART ANTERIEUR

Le document WO 96/38538 enseigne des levures issues de souches de *S. cerevisiae* améliorées présentant une osmotolérance accrue en ce qu'elles produisent des dégagements gazeux (production de CO₂) avantageux dans des pâtes boulangères contenant de fortes concentrations de sucre de 16% à 25% (pourcentages du boulanger). Ce document est muet quant au comportement de ces levures en présence d'inhibiteurs de moisissures, tels que des acides organiques faibles.

Le document US 4318991 divulgue un procédé de production de levures de boulangerie présentant une activité fermentative élevée dans une pâte boulangère même en présence d'agents inhibiteurs de moisissures, tels que des acides organiques faibles comme l'acide acétique ou l'acide propionique. Ce procédé comprend une étape d'ajout d'un tel acide, notamment lors de la multiplication de la levure et ce, dans le dernier stade de sa propagation de sorte à « adapter » la levure audit acide, notamment présent sous forme de propionate de calcium (désigné ci-après par Calpro ou encore ppc) dans les pâtes boulangères. Ce document ne précise pas le comportement des levures ainsi produites dans des pâtes à teneur élevée en sucre.

Un document, au nom de la Demanderesse, US 4346115 décrit un procédé de préparation d'une levure de boulangerie rendue résistante aux acides organiques faibles à partir d'une souche osmotolérante par ajout discontinu de mélasse lors du dernier cycle de sa multiplication.

Un autre document, encore au nom de la Demanderesse EP 1559322, enseigne des souches permettant de produire, après adaptation aux acides faibles, des levures encore plus performantes selon des tests bien connus de l'homme du métier qu'une souche témoin qui a été la référence pendant une vingtaine d'années pour obtenir des levures commerciales performantes sur pâtes sucrées en présence d'inhibiteurs de moisissures.

Outre le fait que l'adaptation, évoquée ci-dessus aux acides faibles, sous forme de propionate de calcium par exemple, au cours de la multiplication de la souche, a un coût certain, ce dernier peut présenter aussi un impact négatif sur l'activité fermentative de la levure, en particulier en présence de pâtes sucrées ou fortement sucrées.

Le point commun désavantageux de ces techniques de l'art antérieur visant à améliorer la résistance de levures de panification aux acides faibles inhibiteurs de moisissures dans les pâtes boulangères sucrées ou non sucrées, en adaptant les souches dont elles sont issues à ces derniers, est de ne conférer aux levures qu'une résistance provisoire/transitoire et non permanente aux acides faibles. L'adaptation consiste à exposer les cellules de levure à une quantité sub-létale d'acide faible lors de leur phase de croissance ; cette exposition préalable permet ensuite aux cellules adaptées de mieux tolérer la présence du même acide faible lorsqu'elles sont mises en conditions de fermentation. Dans certains cas, un acide faible donné peut conférer une adaptation vis-à-vis de la présence ultérieure d'un autre acide faible en phase de fermentation. Ces notions sont exemplifiées dans l'article de Ferreira et coll. (1997) International Journal of Food Microbiology 36, 145-153. Les mécanismes de l'adaptation aux acides faibles ont fait l'objet de plusieurs études. Certaines de ces études ont été rassemblées dans l'article de revue de Piper et coll. (2001) Microbiology 147, 2635-2642. On y apprend que l'adaptation repose sur des mécanismes cellulaires impliquant plusieurs protéines dont certaines pompes membranaires. Ces mécanismes sont non-génétiques et par essence transitoires : ils sont mis en place par les cellules en réaction au stress que représente l'acide faible mais finissent par disparaître en absence de ce stress : les cellules préalablement adaptées reviennent à l'état de cellules non-adaptées.

D'autres procédés bien plus complexes encore, tels ceux décrits dans les documents EP 645094 ou WO 99/51746, enseignent une modification génétique des souches aptes à produire des levures de boulangerie de sorte à les rendre intrinsèquement résistantes aux acides faibles.

La demanderesse, poursuivant ses travaux de recherche de souches toujours plus performantes a constaté qu'une souche particulière issue de sa collection interne de souches osmotolérantes présente un patrimoine génétique tel qu'en l'hybridant ou en la mutant, selon des procédés classiques par ailleurs, il est procuré une souche apte à produire une levure de panification particulièrement performante et ne nécessitant, de manière tout à fait surprenante, aucune adaptation avant son introduction dans des pâtes fortement sucrées contenant un acide faible sous forme de propionate de calcium (désigné par calpro, ou ppc), par exemple.

C'est cette découverte qui est à la base de la présente invention.

### Résumé des objets de la présente invention

Aussi dans son aspect le plus général, la présente invention a trait à une souche de *S*. *cerevisiae* apte à produire une levure de panification présentant une osmotolérance et une résistance intrinsèque aux acides organiques faibles et préparée à partir d'une souche industrielle de *S*. *cerevisiae* déposée le 8 juillet 2010 auprès de la CNCM (Collection Nationale de Cultures de Microorganismes, Institut Curie, 25 rue du docteur Roux, 75724 Paris Cedex 15) sous le numéro I-4341. Plus spécifiquement, la présente invention concerne une souche de *Saccharomyces cerevisiae* susceptible d'être obtenue:
- par un procédé d'hybridation comprenant l'hybridation de la souche industrielle de *Saccharomyces cerevisiae* déposée à la CNCM le 8 juillet 2010 sous le numéro I-4341 avec la souche déposée à la CNCM le 9 février 2011 sous le numéro I-4448, et au moins une étape de sélection de l'hybride obtenu choisie dans le groupe constitué par :
   i. une activité fermentative, mesurée au fermentomètre de Burrows et Harrison dans le test A5' ou dans les tests A5 et A5', l'hybride sélectionné étant tel que son dégagement gazeux est supérieur d'au moins 10% et de préférence de 15 à 25% à celui d'une souche témoin consistant en la souche *Saccharomyces cerevisiae* déposée le 8 juillet 2010 auprès de la CNCM sous le numéro I-4341,
   ii. un temps d'apprêt en No-Time Dough sur pâte contenant 15%, 18%, 23% ou 25% de sucre (pourcentages du boulanger) en présence de 0,4% de propionate de calcium, les hybrides sélectionnés étant aptes à produire des levures de panification par multiplication, en l'absence d'adaptation au propionate de calcium, qui procurent un temps d'apprêt compris entre 85% et 105% et de préférence entre 95% et 105% du temps d'apprêt procuré par une levure de panification témoin produite à partir de la souche industrielle de *Saccharomyces cerevisiae* déposée le 8 juillet 2010 auprès de la CNCM sous le numéro I-4341 par multiplication avec adaptation à la présence de propionate de calcium de celle-ci,
   ou
- par un procédé de mutation aléatoire de la souche industrielle de *Saccharomyces cerevisiae* déposée le 8 juillet 2010 auprès de la CNCM sous le numéro I-4341, et au moins une étape de sélection du mutant obtenu choisie dans le groupe constitué par :
   i. une activité fermentative, mesurée au fermentomètre de Burrows et Harrison dans le test A5", le mutant sélectionné étant tel que son dégagement gazeux est supérieur de 5 à 20% à celui d'une souche témoin consistant en la souche *Saccharomyces cerevisiae* déposée le 8 juillet 2010 auprès de la CNCM sous le numéro I-4341,
   ii. un temps d'apprêt en No-Time Dough sur pâte contenant 15%, 18%, 23% ou 25% de sucre (pourcentages du boulanger) en présence de 0,4% de propionate de calcium, le mutant sélectionné étant apte à produire des levures de panification par multiplication, en l'absence d'adaptation au propionate de calcium, qui procurent un temps d'apprêt compris entre 90% et 105% de celui procuré par une levure de panification témoin produite à partir de la souche industrielle de *Saccharomyces cerevisiae* déposée le 8 juillet 2010 auprès de la CNCM sous le numéro I-4341 par multiplication avec adaptation à la présence de propionate de calcium de celle-ci,
   caractérisée en ce que ladite souche de *Saccharomyces cerevisiae* est apte à produire, par multiplication en l'absence d'adaptation à la présence d'acide(s) faible(s), une levure de panification présentant une osmotolérance et une résistance intrinsèque aux acides organiques faibles.

Dans certains modes de réalisation, ladite souche de *Saccharomyces cerevisiae* est la souche déposée le 11 mai 2010 auprès de la CNCM sous le numéro I-4312.

Dans d'autres modes de réalisation, ladite souche de *Saccharomyces cerevisiae* est la souche déposée le 11 mai 2010 auprès de la CNCM sous le numéro I-4313.

Dans encore d'autres modes de réalisation, ladite souche de *Saccharomyces cerevisiae* est la souche déposée le 8 décembre 2010 auprès de la CNCM sous le numéro I-4409 ou la souche déposée le 8 décembre 2010 auprès de la CNCM sous le numéro I-4410.

A la connaissance de la Demanderesse, c'est la première fois qu'un procédé de production de levure de panification est enseigné qui confère à celle-ci à la fois une osmotolérance et une résistance intrinsèque aux acides faibles en s'affranchissant des procédés coûteux et/ou complexes de l'art antérieur.

La présente invention a encore pour objet une levure de panification susceptible d'être obtenue par multiplication sans adaptation à la présence d'acide(s) faible(s) d'une souche selon la présente invention, ou obtenue par un procédé décrit plus haut.

La Demanderesse a constaté que lorsque lesdites levures de panification sont produites avec adaptation le bénéfice en termes de réduction du proof time est amplifié.

La présente invention a en outre pour objets :
- une pâte boulangère contenant une levure de panification selon la présente invention ;
- ladite pâte boulangère étant de préférence choisie parmi les pâtes dans lesquelles la fermentation est réalisée en présence à la fois d'une pression osmotique due à la présence d'au moins 15% de sucre, de préférence au moins 18% de sucre, de préférence encore au moins 23% de sucre (pourcentages du boulanger) et en présence d'un inhibiteur de moisissures de type acide organique faible, de préférence sous la forme de propionate de calcium.

La présente invention a encore pour objet un procédé de préparation d'un produit cuit de panification par mise en oeuvre de ladite pâte boulangère.

La présente invention a encore pour objet un produit de panification susceptible d'être obtenu par ledit procédé de préparation.

D'autres caractéristiques et avantages vont maintenant être décrits de manière plus détaillée à l'aide d'exemples de réalisation de l'invention donnés à titre purement illustratif et non limitatifs.

### Description détaillée de la présente invention

Dans la présente invention, on entend par levure de panification osmotolérante, une levure qui présente au moins une des propriétés suivantes :
- activité invertase inférieure à 35 unités, de préférence inférieure à 30, et de manière encore plus préférée inférieure à 20, l'unité invertase étant définie comme la production d'une micromole de sucres réducteurs en 5 min par mg de matières sèches de levures à 30°C et à un pH de 4,7 sans plasmolyse de la levure, à savoir une demi-micromole de saccharose inversé (test précisé dans le brevet US 4318929) ;
- passe favorablement l'un au moins des tests suivants au fermentomètre de Burrows et Harrison décrit dans « Journal of Institute of Brewing », vol LXV, No. 1, janvier-février 1959 et sont définis dans le brevet EP 1559322 comme A5, A5', A6 et A6' ;
- passe favorablement l'un au moins des tests de panification suivants par comparaison avec la souche I-4341 adaptée et toujours en présence dans la pâte boulangère de propionate de calcium à 0,4% et de fortes teneurs en sucres, tests appelés dans la présente invention NT15%+, NT18%+, NT23%+, NT25%+: ils correspondent à des mesures du temps d'apprêt en schémas « No-Time Dough » à savoir des procédés directs où il n'y a pratiquement pas de première fermentation entre un pétrissage intensif et la division de la pâte, les pâtons obtenus étant fermentés en moule entre 35°C et 40°C. Cette dernière fermentation, qui est la fermentation essentielle dans un tel procédé est appelée « proof » en anglais. Le proof-time ou temps d'apprêt est défini comme le temps nécessaire pour qu'une pâte de boulangerie atteigne une hauteur donnée dans le moule, correspondant au développement souhaité de la pâte pour qu'elle soit mise au four.

De préférence, une levure osmotolérante selon l'invention possède au moins deux des propriétés telles que définies dans les tests précités.

Dans la présente invention on entend par résistance intrinsèque d'une levure aux acides organiques faibles, présents par exemple sous forme de propionate de calcium, une résistance ni induite, par adaptation par exemple, ni acquise après modification génétique de ladite levure autre que par hybridation ou mutation de la souche dont elle est issue et telles qu'enseignées ci-dessous

Dans la présente invention on entend par souche industrielle, une souche qui contrairement aux souches dites de laboratoire n'est pas forcément haploïde ou diploïde mais dont la ploïdie est souvent plus complexe.

On entend par profil Ty d'une souche, tout profil obtenu par PCR en utilisant des amorces dirigées vers des séquences cibles correspondant à des « cicatrices » génétiques résultant de mouvements de transposons (Ty). Environ 100 copies de ces séquences sont présentes dans le génome de *Saccharomyces cerevisiae,* plus particulièrement dans les séquences proches des gènes codant pour les tRNA. Le nombre et la distribution sur le génome des éléments Ty est variable d'une souche à l'autre et permet de mettre en évidence un polymorphisme important entre souches. Cette technique est maintenant décrite dans la norme DIN CEN/TS 15790 (mars 2009) pour l'identification des levures probiotiques pour l'alimentation animale.

On entend par cartographie de loci de caractères quantitatifs (QTL mapping) une technique qui consiste en la construction de cartes génétiques permettant de localiser des loci (régions du chromosome) impliqués dans la variation de caractères quantitatifs liés à des loci plus facilement identifiables, les marqueurs.

Dans le cadre de la présente invention tous les pourcentages mentionnés dans les tests de sélection, sauf mention du contraire, sont donnés en pourcentages dits du boulanger, bien connus de l'homme du métier, ces pourcentages représentent des pourcentages en masse des ingrédients (par exemple sucres, propionate de calcium etc) présents par rapport à la masse de la farine représentant 100%.

### EXEMPLES

### EXEMPLE 1 : Préparation des hybrides

Les souches hybrides I-4312 et I-4313, objets de la présente invention sont produites par croisements systématiques entre elles de souches de la collection interne de la Demanderesse choisies comme étant osmotolérantes et/ou osmotolérantes et peu sensibles à la présence des acides organiques faibles ou de leurs sels, utilisés comme inhibiteurs de moisissures, telles que celles déposées à la Collection Nationale de Culture de Micro-organismes de l'Institut Pasteur, 25 rue du Docteur Roux, 75724 Paris Cedex 15, France, le 8 juillet 2010 sous le numéro I-4341 et le 9 février 2011 sous le N° I-4448.

Le programme d'hybridation est réalisé selon des techniques classiques comme celles décrites dans les chapitres « Techniques et Protocols » 21 à 23 de l'ouvrage « Methods in Yeast Genetics, A Cold Spring Harbor Laboratory Course Manual, 2000 Edition » de D. Burke, D. Dawson et T. Stearns, Cold Spring Harbor Laboratory Press (ISBN 0-87969-588-9).

### EXEMPLE 2 : Préparation des levures conformes à l'invention à partir des hybrides de l'Exemple 1

De sorte à produire les levures de panification selon l'invention, les souches hybrides telles qu'obtenues à l'Exemple 1, sont mises en culture en fioles ou en fermenteurs dans des milieux connus, mais dépourvus d'acide faible.

En fiole, on pourra utiliser le milieu de culture YPD tel que décrit dans l'appendix A consacré aux milieux de culture de l'ouvrage « Methods in Yeast Genetics, A Cold Spring Harbor Laboratory Course Manual, 2000 Edition » de D. Burke, D. Dawson et T. Stearns, Cold Spring Harbor Laboratory Press (ISBN 0-87969-588-9).

En fermenteur, de façon conventionnelle, les conditions de culture sont telles que le métabolisme de la souche de levure est essentiellement respiratoire et/ou telles qu'il n'y a essentiellement pas de production d'éthanol.

Le terme « essentiellement » signifie que ces conditions sont vérifiées sur la durée totale de la culture, mais qu'il est possible que ponctuellement, ces conditions ne soient pas vérifiées en mode semi-continu, par exemple pendant les première heures de la culture équivalentes à une durée comprise de 1/4 à 1/3 de la durée de culture, et en particulier pendant les 5 premières heures d'une culture en mode semi-continu.

L'expression « le métabolisme de la souche de levure est essentiellement respiratoire » signifie que le carbone est oxydé, et non fermenté, sur la durée totale de la culture, une partie du carbone pouvant être fermentée de manière transitoire.

L'expression « il n'y a essentiellement pas de production d'éthanol » signifie que la concentration en éthanol en fin de culture est inférieure à 1%, de préférence inférieure à 0,1%, plus préférentiellement inférieure à 0,01%, plus préférentiellement inférieure à 0,001%.

L'homme du métier sait comment régler les paramètres de culture d'une souche de levure donnée, afin que les conditions de culture soient telles que le métabolisme de ladite souche est essentiellement respiratoire et/ou telles qu'il n'y a essentiellement pas de production d'éthanol.

La souche de levure selon l'invention est mise en présence du milieu de culture en condition aérobie.

De préférence, la souche de levure selon l'invention est mise en présence du milieu de culture dans un fermenteur adapté à la production de levure. Le volume du fermenteur peut varier de quelques millilitres à plusieurs mètres cube.

Le fermenteur est également appelé réacteur par la suite.

Le fermenteur est de préférence adapté pour une culture en condition aérobie.

La souche de levure est de préférence mise en présence du milieu de culture dans le cadre d'une culture en mode semi-continu et/ou en mode continu.

Par l'expression « culture en mode semi-continu » ou « culture semi-continue » ou « mode semi-continu », appelée également « fed-batch », on désigne ici une culture dans un fermenteur qui est alimenté progressivement par le milieu de culture, mais dont aucun volume de milieu n'est soutiré. Dans un tel procédé, le volume de culture est variable (et croissant) dans le fermenteur. Le débit d'alimentation dans une culture en mode semi-continu est constant ou variable.

Un exemple de culture semi-continue est une culture réalisée dans les conditions décrites dans le livre de référence « Yeast Technology », 2ème édition, 1991, G. Reed et T.W. Nagodawithana, publié par Van Nostrand Reinhold, ISBN 0-442-31892-8.

Le milieu de culture comprend comme constituants le mélange de sucre et les autres éléments nécessaires à la croissance des levures.

Les autres éléments nécessaires à la croissance des levures sont les suivants : au moins une source d'azote, au moins une source de soufre, au moins une source de phosphore, au moins une source de vitamines et/ou au moins une source de minéraux.

Ces autres éléments sont apportés en quantités suffisantes pour ne pas constituer un facteur limitant de la croissance des levures.

La source d'azote est par exemple apportée sous la forme de sulfate d'ammonium, hydroxyde d'ammonium, di-ammonium phosphate, ammoniac, urée et/ou une combinaison de ceux-ci.

La source de soufre est par exemple apportée sous la forme de sulfate d'ammonium, sulfate de magnésium, acide sulfurique et/ou une combinaison de ceux-ci.

La source de phosphore est par exemple apportée sous la forme d'acide phosphorique, phosphate de potassium, di-ammonium phosphate, mono-ammonium phosphate, et/ou une combinaison de ceux-ci.

La source de vitamines est par exemple apportée sous la forme de mélasse, hydrolysat de levure, solution de vitamine pure ou d'un mélange de vitamines pures et/ou une combinaison de ceux-ci.

La source de vitamines apporte à la levure l'ensemble des vitamines dans des quantités au moins équivalentes à celles recommandées dans les ouvrages de référence. Plusieurs sources de vitamines peuvent être associées.

La source de minéraux est par exemple apportée sous la forme de mélasse, un mélange de sels minéraux et/ou leur combinaison.

La source de minéraux apporte à la levure l'ensemble des macroéléments et oligoéléments dans des quantités au moins équivalentes à celles recommandées dans les ouvrages de référence. Plusieurs sources de minéraux peuvent être associées.

Une même substance peut apporter plusieurs éléments différents.

Les conditions de production de la levure de boulangerie en fermenteur sont explicitées en détail par A. Loiez dans le chapitre « Production de levure de panification par biotechnologie » des Techniques de l'Ingénieur, Référence J6013 du 10 mars 2003.

Le même milieu de culture des hybrides selon l'invention est utilisé pour la souche de référence I-4341, à laquelle ils vont être comparés, mais en procédant pour ce qui la concerne à une étape supplémentaire dite d'adaptation selon la technique décrite dans les brevets US 4318991 ou US 4346115 ou selon l'enseignement combiné de ces deux documents.

Le procédé de préparation de la levure de panification comprend au moins les deux premières étapes de l'ensemble des étapes suivantes consistant à :
- multiplier une souche pure en conditions semi-aérobies puis aérobies,
- séparer par centrifugation la levure ainsi produite de son milieu de culture, de sorte à procurer une « crème de levure » liquide contenant de 14% à 25% de matières sèches ou plus si la crème de levure est mélangée à des produits osmotiques,
- filtrer la crème de levure liquide ainsi obtenue, de préférence sur un filtre rotatif sous vide et procurer une levure fraîche déshydratée contenant de 26 à 35% de matières sèches,
- malaxer ladite levure ainsi obtenue, soit sous forme de pains de levure fraîche, soit sous forme de levure fraîche émiettée à environ 30% de matières sèches, soit encore sous forme de particules, de préférence des granules, si la levure est destinée à être séchée,
- sécher de manière ménagée, dans un courant d'air chaud, par exemple par fluidisation, des particules de levure obtenues après extrusion,
- emballer avant commercialisation.

### EXEMPLE 3 : Comparaison des performances des levures non adaptées selon l'invention avec la souche témoin adaptée

### Exemple 3.1 : Présentation des tests

### 3.1.1. Tests au fermentomètre

Les tests sont réalisés à l'aide du fermentomètre de Burrows et Harrison décrit dans la « Journal of Institute of Brewing », vol. LXV, No. 1, janvier-février 1959 et sont définis de la manière suivante :

### Test A5

Une suspension de levure est préparée de la façon suivante :
1. 200 mg de matière sèche de la levure à tester,
2. 5 ml d'une solution à 108g/L de NaCl et 16g/L de (NH₄)₂SO₄, et
3. Mettre à volume à 20ml avec de l'eau distillée.

15ml de la suspension ci-dessus (soit 150 mg de matière sèche levure) sont équilibrés à une température de 30°C pendant 15 minutes. A cette suspension est ajouté un mélange de 20g de farine et 4g de saccharose préalablement équilibrés une nuit à 30°C. L'ensemble est homogénéisé pendant une durée de 35 secondes.

La pâte formée est incubée dans un récipient fermé hermétiquement placé à 30°C. Le dégagement gazeux (exprimé en ml sous 760 mm Hg) est enregistré sur une durée totale de 120 minutes (cette durée peut être adaptée).

### Test A5'

Protocole identique au A5 sauf que :
1. On ajoute 500µl d'une solution à 16% (w/v) de propionate de calcium (calpro) par-dessus le mélange farine+sucre juste avant la phase de mélange de 35 secondes.

### Test A5"

Protocole identique au A5 sauf que :
1. On ajoute 1 ml d'une solution à 16% (w/v) de propionate de calcium par-dessus le mélange farine+sucre juste avant la phase de mélange de 35 secondes.

### Test A6

Protocole identique au PS4g sauf que :
1. La suspension de levure est préparée avec 400 mg de matière sèche (au lieu de 200 mg), ce qui entraîne que c'est une quantité de 300 mg de matière sèche levure qui est mise en oeuvre dans le test.
2. Le mélange farine + sucre est composé de 25g de farine (au lieu de 20g) et 6.5g de saccharose (au lieu de 4g).

### Test A51 et A51' (avec propionate de Calcium)

### Pour levure fraîche

Une suspension de levure est préparée de la façon suivante :
1. 400 mg de matière sèche de la levure à tester,
2. Délayer dans le volume d'eau distillée nécessaire pour atteindre un volume total d'eau de 6.8 ml en tenant compte de l'eau apportée par la levure fraîche (typiquement 70% du poids d'une levure pressée),
3. Equilibrer à 30°C pendant 22 minutes, et
4. Ajouter 4.5 ml d'une solution à 73.6g/kg de NaCl et homogénéiser.

A la suspension ci-dessus on ajoute 20g de farine et 3g de saccharose. L'ensemble est mélangé pendant une durée de 35 secondes.

Le dégagement gazeux (exprimé en ml sous 760 mm Hg) est enregistré sur une durée totale de 120 minutes (cette durée peut être adaptée). La mesure démarre 36 minutes après le début du protocole.

Ce protocole existe dans une variante incluant la présence de propionate de Calcium. Dans ce cas, à l'étape 4 de la préparation de la suspension de levure, la solution à 73.6g/kg de NaCl contient en plus 17.4g/kg de propionate de Calcium.

### Pour levure sèche

Mélanger 20g de farine, 3g de saccharose et 400 mg de matière sèche de la levure à tester. Equilibrer à 30°C pendant 12 minutes.

Ajouter 6.8 ml d'eau distillée et 4.5 ml d'une solution à 73.6g/kg de NaCl et homogénéiser pendant 35 secondes.

36 minutes après l'ajout de l'eau distillée, procéder à une seconde homogénéisation identique à la première.

Le dégagement gazeux (exprimé en ml sous 760 mm Hg) est enregistré sur une durée totale de 120 minutes (cette durée peut être adaptée). La mesure démarre 60 minutes après le début du protocole.

### Test A61 et A61' (avec propionate de Calcium)

### Pour levure fraîche

Une suspension de levure est préparée de la façon suivante :
1. 700 mg de matière sèche de la levure à tester,
2. Délayer dans le volume d'eau distillée nécessaire pour atteindre un volume total d'eau de 7.5 ml en tenant compte de l'eau apportée par la levure fraîche (typiquement 70% du poids d'une levure pressée),
3. Equilibrer à 30°C pendant 22 minutes, et
4. Ajouter 5.75 ml d'une solution à 73.6g/kg de NaCl et homogénéiser.

A la suspension ci-dessus on ajoute 25g de farine et 6.5g de saccharose.

L'ensemble est mélangé pendant une durée de 35 secondes.

Le dégagement gazeux (exprimé en ml sous 760 mm Hg) est enregistré sur une durée totale de 120 minutes (cette durée peut être adaptée). La mesure démarre 36 minutes après le début du protocole.

Ce protocole existe dans une variante incluant la présence de propionate de Calcium. Dans ce cas, à l'étape 4 de la préparation de la suspension de levure, la solution à 73.6g/kg de NaCl contient en plus 17.4g/kg de propionate de Calcium.

### Pour levure sèche

Mélanger 25g de farine, 6.5g de saccharose et 700mg de matière sèche de la levure à tester. Equilibrer à 30°C pendant 12 minutes.

Ajouter 7.5 ml d'eau distillée et 5.75 ml d'une solution à 73.6g/kg de NaCl et homogénéiser pendant 35 secondes.

36 minutes après l'ajout de l'eau distillée, procéder à une seconde homogénéisation identique à la première.

Le dégagement gazeux (exprimé en ml sous 760 mm Hg) est enregistré sur une durée totale de 120 minutes (cette durée peut être adaptée). La mesure démarre 60 minutes après le début du protocole.

### 3.1.2. Tests de panification, 4 recettes sont testées :

- la recette PT1 contenant 15% en masse (pourcentage du boulanger) de saccharose et 0,4% en masse (pourcentage du boulanger) de propionate de calcium ;
- la recette PT2 contenant 18% en masse (pourcentage du boulanger) de saccharose et 0,4% en masse (pourcentage du boulanger) de propionate de calcium ;
- la recette PT3 contenant 23% en masse (pourcentage du boulanger) de saccharose et 0,4% en masse (pourcentage du boulanger) de propionate de calcium ;
- la recette PT4 contenant 25% en masse (pourcentage du boulanger) de saccharose et 0,4% en masse (pourcentage du boulanger) de propionate de calcium.

Dans les tests 1 à 4, on mesure, dans un procédé de panification donné et avec des recettes données, l'écart en Proof Time entre, d'une part, une levure sèche obtenue avec la souche à évaluer, et, d'autre part, une levure sèche obtenue avec une souche témoin « T ». La performance est mesurée en pourcentages d'écart entre la souche testée et la souche témoin. Un écart négatif correspond à une performance supérieure à celle de la souche témoin.

Les recettes utilisées dans les tests et exprimées en pourcentage du boulanger sont données dans le tableau suivant. Ce tableau comprend également le détail des différents protocoles utilisés.

| | | ***No Time 15%+*** | ***No Time 18%+*** | ***Rotimani 23%+*** | ***No Time 25%+*** |
|---|---|---|---|---|---|
| **TEST** | | **PT1** | **PT2** | **PT3** | **PT4** |
| **FORMULES** | | **Recette 1** | **Recette 2** | **Recette 3** | **Recette 4** |
| Farine | | 100% | 100% | 100% | 100% |
| Eau | | 54% | 52% | 45% | 50% |
| Saccharose | | 15% | 18% | 23% | 25% |
| Sel | | 1,7% | 1% | 1,5% | 1,7% |
| Levure sèche | | 1,5% | 1% | 2,3% | 2% |
| Matière Grasse | | 7,5% | 5% | 10% | 7,5% |
| Améliorant | | - | 0,5% | 0,3% | - |
| | | 1% | - | - | 1% |
| Poudre de lait | | - | 4% | 3% | - |
| Oeufs | | - | 6% | 10% | - |
| Calpro | | 0,4% | 0,4% | 0,3% | 0,4% |
| | | | | | |

| **TEMPERATURES** | | **Recette 1** | **Recette 2** | **Recette 3** | **Recette 4** |
|---|---|---|---|---|---|
| Local+Farine+Eau | | 64°C | 70°C | 70°C | 64°C |
| Bain-marie Pétrins | | 22,5°C | 23,5°C | 24,5°C | 22,5°C |
| Pâte | | 27°C | 28°C | 29°C | 27°C |
| | | | | | |

| **DIAGRAMME** | | **Recette 1** | **Recette 2** | **Recette 3** | **Recette 4** |
|---|---|---|---|---|---|
| Pétrissage | Mac Duffy | L5 R5 H4 | L1 +MG1 L1 H4 | L1 +MG1 L1 H5,5 | L5 R5 H4 |
| Pointage Masse | | 10 min | 15 min | 10 min | 10 min |
| Division | | 320 g | 320 g | 320 g | 320 g |
| Boulage - Détente | | | | | |
| Temps Fin pétrissage - début façonnage | | 25 min | 50 min | 25 min | 25 min |
| Façonnage | Façonneuse US | | | | |
| Apprêt | Etuve Forma | 35°C 90%HRE | 35°C 90%HRE | 35°C 90%HRE | 35°C 90%HRE |
| Cuisson | Four Reed | 21 min 190°C | 21 min 190°C | 21 min 190°C | 21 min 190°C |

### DETAIL DU MODE OPERATOIRE :

Le protocole d'essai appliqué aux différentes recettes ci-dessus dans les tests PT1 à PT4 est le suivant :
1. Peser les divers ingrédients.
2. Mesurer la température ambiante et la température de la farine.
3. Régler la température de l'eau de manière à obtenir une température de pâte, à +/-0.5°C, de 27°C pour les recettes 1 et 4 ; 28°C pour la recette 2 et 29°C pour la recette 3.
4. Placer les ingrédients dans une cuve Mac Duffy® d'un pétrin Hobart A200®, dont la double enveloppe est préalablement thermostatée avec une eau à 22.5°C (recettes 1 et 4) ou 23.5°C (recette 2) ou 24.5 °C (recette 3).
5. Mélanger les ingrédients secs en première vitesse pendant 1 minute
6. Le diagramme de pétrissage dépend de la recette employée :
   - recettes 1 et 4 :
      - la matière grasse est incorporée après le prémélange à sec,
      - l'eau de coulage est ajoutée,
      - malaxage 5 minutes en première vitesse (L5),
      - repos 5 minutes (R5), et
      - pétrissage 4 minutes en seconde vitesse (H4),
   - recette 2 :
      - l'eau de coulage est ajoutée après le prémélange à sec,
      - malaxage 1 minute en première vitesse (L1),
      - repos 1 minute pour l'ajout de la matière grasse (+MG1),
      - malaxage 1 minute en première vitesse (L1), et
      - pétrissage 4 minutes en seconde vitesse (H4)
   - recette 3 :
      - l'eau de coulage est ajoutée après le prémélange à sec,
      - malaxage 1 minute en première vitesse (L1),
      - repos 1 minute pour l'ajout de la matière grasse (+MG1),
      - malaxage 1 minute en première vitesse (L1), et
      - pétrissage 5 minutes 30 secondes, en seconde vitesse (H5.5)
7. Vérifier la température de la pâte obtenue en fin de pétrissage,
8. Pointage de la masse à 23°C pendant 10 minutes (recettes 1, 3 et 4) ou 15 minutes (recette 2),
9. Division en pâtons de 320g,
10. Bouler peu serré et couvrir,
11. Laisser reposer 10 minutes (recettes 1, 3 et 4) ou 30 minutes (recette 2),
12. Façonnage de la pâte,
13. Mise en moule des pâtons de 320 g (dimensions : base du moule de 185x75 mm ; haut du moule de 200x90 mm ; hauteur du moule de 75 mm),
14. Détermination du temps d'apprêt, dénommé Proof Time, dans un incubateur Stéricult® à 35°C et 90% d'humidité relative. Le Proof Time est le temps entre la mise en place des moules dans l'incubateur et le moment où la pâte atteint une hauteur de 85 mm dans le moule, et
15. Cuisson dans un four à balancelle REED® 21 minutes à 190°C.

### Exemple 3.2 : Résultats

**Tableau 1 : tests au fermentomètre. Ecarts hybride selon l'invention vs parent I-4341**

| **Biomasse produite en fiole, évaluation sur levure fraîche** | | | | |
|---|---|---|---|---|
| Test | **Parent 1 I-4341 sans adaptation** | **Parent 2 I-4448 sans adaptation** | **I-4312 sans adaptation** | **I-4313 sans adaptation** |
| **A5** | 122 | 120 | 137 | 132 |
| **A5'** | 92 | 98 | 114 | 115 |
| Gain /parents | | | 17 & 24% | 18 & 24% |
| | | | | |

| **Biomasse produite en fermenteur 7L, évaluation sur levure fraîche** | | | | |
|---|---|---|---|---|
| Test | **I-4341 sans adaptation** | **I-4341 avec adaptation** | **I-4312 sans adaptation** | **I-4313 sans adaptation** |
| **A5** | 96 | 99 | 121 | 126 |
| **A5'** | 79 | 108 | 114 | 107 |
| **A61** | 139 | 179 | 209 | 225 |
| **A61'** | 88 | 105 | 121 | 131 |
| | | | | |

| **Biomasse produite en fermenteur 20L, évaluation sur levure sèche** | | | | |
|---|---|---|---|---|
| Test | **I-4341 sans adaptation** | **I-4341 avec adaptation** | **I-4312 sans adaptation** | **I-4313 sans adaptation** |
| **A5** | 102 | 105 | 105 | 97 |
| **A5'** | 99 | 107 | 90 | 80 |
| **A51** | 159 | 214 | 173 | 209 |
| **A51'** | 124 | 160 | 107 | 134 |
| **A61** | 167 | 232 | 169 | 241 |
| **A61'** | 95 | 114 | 97 | 125 |

**Tableau 2 : test en No-Time Dough hybride selon l'invention vs la levure obtenue à partir de la souche non adaptée déposée le 8 juillet 2010 à la CNCM sous le N°I-4342.**

| **Biomasse produite en fermenteur 20L, évaluation sur levure sèche** | | |
|---|---|---|
| Test | **I-4342 sans adaptation** | **I-4313 sans adaptation** |
| PT1 | T | -14% |
| PT2 | T | -9% |
| PT3 | T | -17% |

**Tableau 3 : No-Time Dough hybrides selon l'invention vs témoin adaptée (= I-4341adaptée)**

| **Biomasse produite en fermenteur 20L, évaluation sur levure sèche** | | |
|---|---|---|
| Test | **I-4341 avec adaptation** | **I-4313 sans adaptation** |
| **PT2** | T | 1% |
| **PT3** | T | -5% |
| **PT4** | T | 3% |

Conclusion : les levures non adaptées selon l'invention se comportent de manière sensiblement identique voire meilleure par comparaison à une levure témoin adaptée, produite par croisement d'une des meilleures souches osmotolérantes disponibles dans la collection interne de la Demanderesse et déposée sous le numéro I-4341 et d'une souche du commerce peu sensible aux acides faibles.

Ces résultats sont surprenants car il n'était absolument pas prévisible de cumuler les avantages préexistants des deux parents en hybridant des souches industrielles, surtout en dépassant de 15 % à 25 % les valeurs de dégagement gazeux de leurs deux parents.

La présente invention procure ainsi des hybrides aptes à produire des levures de panification à moindre coût tout en étant aussi performantes dans des pâtes boulangères fortement sucrées et en présence d'inhibiteurs de moisissures comme le propionate de calcium.

### EXEMPLE 4 : Préparation des mutants I-4409 et I-4410

La souche I-4341 a été soumise à une mutagenèse par exposition aux rayonnements ultraviolets. Une population de mutants a été évaluée dans les tests A5 et A5' après production en fiole. Les souches I-4409 et I-4410 ont été sélectionnées au départ de cette population.

### EXEMPLE 5 : Préparation de la levure à partir des mutants de l'Exemple 4 (idem exemple 2)

### EXEMPLE 6: Comparaison des performances avec une levure témoin

**Biomasse produite en fiole, évaluation sur levure fraîche**

| Test | **I-4341 sans adaptation** | **I-4409 sans adaptation** | **I-4410 sans adaptation** |
|---|---|---|---|
| **A5** | 121 | 117 | 126 |
| **A5"** | 88 | 105 | 96 |
| Gain / I-4341 dans A5" (%) | | 19 | 9 |

**Biomasse produite en fermenteur 20L, évaluation sur levure sèche**

| Test | **I-4341 avec adaptation** | **I-4409 sans adaptation** | Ecart (%) |
|---|---|---|---|
| **A5** | 101 | 101 | 0 |
| **A5'** | 104 | 100 | -4 |
| **A51** | 104 | 114 | 10 |
| **A51'** | 136 | 159 | 17 |

| Test | **I-4341 avec adaptation** | **I-4410 sans adaptation** | Ecart (%) |
|---|---|---|---|
| **A5** | 96 | 106 | 10 |
| **A5'** | 101 | 97 | **-4** |

Les résultats de panification obtenus avec ces levures sont ci-dessous (tests PT2 et PT3)

| Test | **I-4341 avec adaptation** | **I-4410 sans adaptation** | **I-4410 avec adaptation** |
|---|---|---|---|
| **PT2** | T | 2% | -11% |
| **PT3** | T | 2% | -4% |

| Test | **I-4341 avec adaptation** | **I-4409 sans adaptation** |
|---|---|---|
| **PT1** | T | **3%** |
| **PT2** | T | **5%** |
| **PT3** | T | **-1%** |

## Revendications

1. Souche de *Saccharomyces cerevisiae* susceptible d'être obtenue:
- par un procédé d'hybridation comprenant l'hybridation de la souche industrielle de *Saccharomyces cerevisiae* déposée à la CNCM le 8 juillet 2010 sous le numéro 1-4341 avec la souche déposée à la CNCM le 9 février 2011 sous le numéro 1-4448, et au moins une étape de sélection de l'hybride obtenu choisie dans le groupe constitué par :
i. une activité fermentative, mesurée au fermentomètre de Burrows et Harrison dans le test A5' ou dans les tests A5 et A5', l'hybride sélectionné étant tel que son dégagement gazeux est supérieur d'au moins 10% et de préférence de 15 à 25% à celui d'une souche témoin consistant en la souche *Saccharomyces cerevisiae* déposée le 8 juillet 2010 auprès de la CNCM sous le numéro 1-4341,
ii. un temps d'apprêt en No-Time Dough sur pâte contenant 15%, 18%, 23% ou 25% de sucre (pourcentages du boulanger) en présence de 0,4% de propionate de calcium, les hybrides sélectionnés étant aptes à produire des levures de panification par multiplication, en l'absence d'adaptation au propionate de calcium, qui procurent un temps d'apprêt compris entre 85% et 105% et de préférence entre 95% et 105% du temps d'apprêt procuré par une levure de panification témoin produite à partir de la souche industrielle de *Saccharomyces cerevisiae* déposée le 8 juillet 2010 auprès de la CNCM sous le numéro 1-4341 par multiplication avec adaptation à la présence de propionate de calcium de celle-ci,
ou
- par un procédé de mutation aléatoire de la souche industrielle de *Saccharomyces cerevisiae* déposée le 8 juillet 2010 auprès de la CNCM sous le numéro 1-4341, et au moins une étape de sélection du mutant obtenu choisie dans le groupe constitué par :
i. une activité fermentative, mesurée au fermentomètre de Burrows et Harrison dans le test A5", le mutant sélectionné étant tel que son dégagement gazeux est supérieur de 5 à 20% à celui d'une souche témoin consistant en la souche *Saccharomyces cerevisiae* déposée le 8 juillet 2010 auprès de la CNCM sous le numéro 1-4341,
ii. un temps d'apprêt en No-Time Dough sur pâte contenant 15%, 18%, 23% ou 25% de sucre (pourcentages du boulanger) en présence de 0,4% de propionate de calcium, le mutant sélectionné étant apte à produire des levures de panification par multiplication, en l'absence d'adaptation au propionate de calcium, qui procurent un temps d'apprêt compris entre 90% et 105% de celui procuré par une levure de panification témoin produite à partir de la souche industrielle de *Saccharomyces cerevisiae* déposée le 8 juillet 2010 auprès de la CNCM sous le numéro 1-4341 par multiplication avec adaptation à la présence de propionate de calcium de celle-ci,
**caractérisée en ce que** ladite souche de *Saccharomyces cerevisiae* est apte à produire, par multiplication en l'absence d'adaptation à la présence d'acide(s) faible(s), une levure de panification présentant une osmotolérance et une résistance intrinsèque aux acides organiques faibles.

2. Souche de *Saccharomyces cerevisiae* selon la revendication 1 déposée le 11 mai 2010 auprès de la CNCM sous le numéro 1-4312.

3. Souche de *Saccharomyces cerevisiae* selon la revendication 1 déposée le 11 mai 2010 auprès de la CNCM sous le numéro 1-4313.

4. Souche de *Saccharomyces cerevisiae* selon la revendication 1 déposée auprès de la CNCM sous le numéro 1-4409.

5. Souche de *Saccharomyces cerevisiae* selon la revendication 1 déposée auprès de la CNCM sous le numéro 1-4410.

6. Levure de panification susceptible d'être obtenue par multiplication sans adaptation à la présence d'acide(s) faible(s) d'une souche selon l'une quelconque des revendications 2 à 5 ou obtenue par un procédé défini dans la revendication 1.

7. Pâte boulangère contenant une levure de panification selon la revendication 6.

8. Pâte boulangère selon la revendication 7, **caractérisée en ce qu'**elle est choisie parmi les pâtes dans lesquelles la fermentation est réalisée en présence à la fois d'une pression osmotique due à la présence d'au moins 15% de sucre, de préférence au moins 18% de sucre, de préférence encore au moins 23% de sucre et en présence d'un inhibiteur de moisissures de type acide organique faible, de préférence sous forme de propionate de calcium, les pourcentages étant des pourcentages de boulanger.

9. Procédé de préparation d'un produit cuit de panification par mise en oeuvre d'une pâte boulangère selon la revendication 7 ou la revendication 8.

10. Produit de panification susceptible d'être obtenu par le procédé selon la revendication 9.

## Patentansprüche

1. Saccharomyces cerevisiae-Stamm, der erhalten werden kann:
- durch ein Verfahren zur Hybridisierung, welches die Hybridisierung des bei der CNCM am 8. Juli 2010 unter der Nummer I-4341 hinterlegten industriellen Saccharomyces cerevisiae-Stamms umfasst, mit dem bei der CNCM am 9. Februar 2011 unter der Nummer I-4448 hinterlegten Stamm, und wenigstens einen Schritt der Selektion des erhaltenen Hybrids, gewählt aus der Gruppe, umfassend:
i. eine mittels des Fermentometers von Burrows und Harrison im A5'-Test oder den A5- und A5'-Tests gemessene fermentative Aktivität, wobei das gewählte Hybrid derart ist, dass seine Gasfreisetzung wenigstens um 10% und bevorzugt um 15 bis 25% höher ist als diejenige eines Kontrollstamms, der aus dem bei der CNCM am 8. Juli 2010 unter der Nummer I-4341 hinterlegten Saccharomyces cerevisiae-Stamm besteht,
ii. eine Gärungszeit als No-Time Dough bei Teig, welcher 15%, 18%, 23% oder 25% Zucker enthält (Bäckerei-Prozentsätze) in Gegenwart von 0,4% Calciumpropionat, wobei die gewählten Hybride in der Lage sind, Backhefen durch Vermehrung in Abwesenheit von Adaptierung mit Calciumpropionat herzustellen, die eine Gärungszeit zwischen 85% und 105%, bevorzugt zwischen 95% und 105% der Gärungszeit durch eine Referenzbackhefe ermöglichen, die ausgehend von dem am 8. Juli 2010 unter der Nummer I-4341 hinterlegten industriellen Saccharomyces cerevisiae-Stamm durch dessen Vermehrung mit Adaptierung in Gegenwart von Calciumpropionat hergestellt wurde,
oder
- durch ein Verfahren der zufälligen Mutation des bei der CNCM am 8. Juli 2010 unter der Nummer I-4341 hinterlegten industriellen Saccharomyces cerevisiae-Stamms, und wenigstens einen Selektionsschritt der erhaltenen Mutante, gewählt aus der Gruppe, umfassend:
i. eine mittels des Fermentometers von Burrows und Harrison im A5"-Test gemessene fermentative Aktivität, wobei die gewählte Mutante derart ist, dass ihre Gasfreisetzung um 5 bis 20% höher ist als diejenige eines Kontrollstamms, der aus dem bei der CNCM am 8. Juli 2010 unter der Nummer I-4341 hinterlegten Saccharomyces cerevisiae-Stamm besteht,
ii. eine Gärungszeit als No-Time Dough bei Teig, welcher 15%, 18%, 23% oder 25% Zucker enthält (Bäckerei-Prozentsätze) in Gegenwart von 0,4% Calciumpropionat, wobei die gewählte Mutante in der Lage ist, Backhefen durch Vermehrung in Abwesenheit von Adaptierung mit Calciumpropionat herzustellen, die eine Gärungszeit zwischen 90% und 105% der Gärungszeit durch eine Referenzbackhefe ermöglichen, die ausgehend von dem am 8. Juli 2010 unter der Nummer I-4341 hinterlegten industriellen Saccharomyces cerevisiae-Stamm durch dessen Vermehrung mit Adaptierung in Gegenwart von Calciumpropionat hergestellt wurde, **dadurch gekennzeichnet, dass** der Saccharomyces cerevisiae-Stamm in der Lage ist, durch Vermehrung in Abwesenheit von Adaptierung in Gegenwart von schwacher Säure/schwachen Säuren eine Backhefe herzustellen, die osmotolerant ist und eine intrinsische Resistenz gegenüber schwachen organischen Säuren aufweist.

2. Saccharomyces cerevisiae-Stamm nach Anspruch 1, der bei der CNCM am 11. Mai 2010 unter der Nummer I-4312 hinterlegt wurde.

3. Saccharomyces cerevisiae-Stamm nach Anspruch 1, der bei der CNCM am 11. Mai 2010 unter der Nummer I-4313 hinterlegt wurde.

4. Saccharomyces cerevisiae-Stamm nach Anspruch 1, der bei der CNCM unter der Nummer I-4409 hinterlegt wurde.

5. Saccharomyces cerevisiae-Stamm nach Anspruch 1, der bei der CNCM unter der Nummer I-4410 hinterlegt wurde.

6. Backhefe, die durch Vermehrung ohne Adaptierung in Gegenwart von schwacher Säure/schwachen Säuren eines Stamms nach einem der Ansprüche 2 bis 5 oder durch ein in Anspruch 1 beschriebenes Verfahren erhalten werden kann.

7. Backteig, welcher eine Backhefe nach Anspruch 6 umfasst.

8. Backteig nach Anspruch 7, **dadurch gekennzeichnet, dass** er gewählt ist aus den Teigen, bei welchen die Gärung bei gleichzeitigem Vorliegen eines osmotischen Drucks aufgrund der Gegenwart von wenigstens 15% Zucker, bevorzugt wenigstens 18% Zucker, ferner bevorzugt wenigstens 23% Zucker und in Gegenwart eines Fungistatikums vom Typ schwache organische Säure, bevorzugt in Form von Calciumpropionat erfolgt, wobei die Prozentsätze Bäcker-Prozentsätze sind.

9. Zubereitungsverfahren für ein gebackenes Brotprodukt durch Ausführung eines Backteigs nach Anspruch 7 oder Anspruch 8.

10. Brotprodukt, welches durch das Verfahren nach Anspruch 9 erhalten werden kann.

## Claims

1. Saccharomyces *cerevisiae* strain which can be obtained:
- by means of a hybridization method comprising hybridization of the industrial strain of *Saccharomyces cerevisiae* deposited at the CNCM [French National Collection of Microorganism Cultures] on July 8, 2010 under number I-4341 with the strain deposited at the CNCM on February 9, 2011 under number 1-4448, and at least one step of selection of the hybrid obtained, chosen from the group consisting of:
i. a fermentative activity, measured using a Burrows and Harrison fermentometer in test A5' or in tests A5 and A5', the hybrid selected being such that its gas release is at least 10% and preferably from 15% to 25% greater than that of a control strain consisting of the *Saccharomyces cerevisiae* strain deposited on July 8, 2010 with the CNCM under number 1-4341,
ii. a proof time in a No-Time Dough scheme on dough containing 15%, 18%, 23% or 25% of sugar (baker's percentages) in the presence of 0.4% of calcium propionate, the hybrids selected being capable of producing by multiplication, in the absence of adaptation to calcium propionate, baker's yeasts which exhibit a proof time of between 85% and 105%, and preferably between 95% and 105%, of the proof time exhibited by a control baker's yeast produced from the industrial strain of *Saccharomyces cerevisiae* deposited on July 8, 2010 with the CNCM under number 1-4341 by multiplication with adaptation to the presence of calcium propionate of said strain,
or
- by means of a random mutation method of the industrial strain of Saccharomyces cerevisiae deposited on July 8, 2010 with the CNCM under number 1-4341, and at least one step of selection of the mutant obtained, chosen from the group consisting of:
i. a fermentative activity, measured using a Burrows and Harrison fermentometer in test A5", the mutant selected being such that its gas release is from 5 to 20% greater than that of a control strain consisting of the *Saccharomyces cerevisiae* strain deposited on July 8, 2010 with the CNCM under number 1-4341,
ii. a proof time in a No-Time Dough scheme on dough containing 15%, 18%, 23% or 25% of sugar (baker's percentages) in the presence of 0.4% of calcium propionate, the mutant selected being capable of producing, by multiplication in the absence of adaptation to calcium propionate, baker's yeasts which exhibit a proof time of between 90% and 105% of that exhibited by a control baker's yeast produced from the industrial strain of *Saccharomyces cerevisiae* deposited on July 8, 2010 with the CNCM under number 1-4341 by multiplication with adaptation to the presence of calcium propionate of said strain,
**characterized in that** said *Saccharomyces cerevisiae* strain is capable of producing, by multiplication without adaptation to the presence of weak acid(s), a baker's yeast which exhibits osmotolerance and an intrinsic resistance to weak organic acids.

2. Saccharomyces *cerevisiae* strain according to Claim 1, deposited on May 11, 2010 with the CNCM under number 1-4312.

3. Saccharomyces *cerevisiae* strain according to Claim 1, deposited on May 11, 2010 with the CNCM under number 1-4313.

4. *Saccharomyces cerevisiae* strain according to Claim 1, deposited with the CNCM under number 1-4409.

5. *Saccharomyces cerevisiae* strain according to Claim 1, deposited with the CNCM under number 1-4410.

6. Baker's yeast which can be obtained by multiplication, without adaptation to the presence of weak acid(s), of a strain according to any one of Claims 2 to 5 or obtained by means of a method defined in Claim 1.

7. Baker's dough containing a baker's yeast according to Claim 6.

8. Baker's dough according to Claim 7, **characterized in that** it is chosen from doughs in which the fermentation is carried out both in the presence of an osmotic pressure due to the presence of at least 15% of sugar, preferably at least 18% of sugar and more preferably at least 23% of sugar and in the presence of a mold inhibitor of weak organic acid type, preferably in the form of calcium propionate, the percentages being expressed as baker's percentages.

9. Method for preparing a baked bread product by using a baker's dough according to Claim 7 or Claim 8.

10. Bread product which can be obtained by means of the method according to Claim 9.
